Europäisches Patentamt

(19) European Patent Office (11) Veröffentlichungsnummer: **0 170 670**

Office européen des brevets **B1**

(12) # EUROPÄISCHE PATENTSCHRIFT

(45) Veröffentlichungstag der Patentschrift:
12.08.87

(21) Anmeldenummer: **85900667.8**

(22) Anmeldetag: **29.01.85**

(86) Internationale Anmeldenummer:
**PCT/EP 85/00025**

(87) Internationale Veröffentlichungsnummer:
**WO 85/03278 (01.08.85 Gazette 85/17)**

(51) Int. Cl.⁴: **B 65 G 47/14**

(54) **TRANSPORTEINRICHTUNG FÜR TABLETTEN.**

(30) Priorität: **30.01.84 DE 8402581 U**

(43) Veröffentlichungstag der Anmeldung:
**12.02.86 Patentblatt 86/7**

(45) Bekanntmachung des Hinweises auf die Patenterteilung:
**12.08.87 Patentblatt 87/33**

(84) Benannte Vertragsstaaten:
**CH DE FR GB LI**

(56) Entgegenhaltungen:
**FR-A-2 263 181**
**US-A-2 955 696**

**Transactions of the ASME, Vol. 99, No. 2, May 1977, New York (US). L.E. Murch et al.: "Analysis of Feeding and Orienting Systems for Automatic Assembly", pages 308-312, see figure 10**

(73) Patentinhaber: **Erweka Apparatebau GmbH, Ottostrasse 20- 22, D-6056 Heusenstamm (DE)**

(72) Erfinder: **KAMINSKI, Reiner, Wilhelm- Leuchner- Str. 4, D-6056 Rodgau (DE)**

(74) Vertreter: **Patentanwälte Kirschner & Grosse, Herzog- Wilhelm- Strasse 17, D-8000 München 2 (DE)**

EP 0 170 670 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Transporteinrichtung für Tabletten gemäß dem Oberbegriff des Patentanspruchs 1.

Derartige Transporteinrichtungen werden beispielsweise in Tabletten-Prüfgeräten oder Verpackungseinrichtungen für Tabletten benötigt.

In jedem Falle ist es erforderlich, daß die Transporteinrichtung jeweils nur eine Tablette der Prüfstation, beispielsweise einer Waage, oder der Verpackungseinrichtung zuführt. Würde nämlich die Transporteinrichtung zwei Tabletten auf einmal beispielsweise einer Waage oder einer Verpackungsstation für Einzelverpackung zuführen, würde ein falsches Gewicht bestimmt bzw. es würden zwei Tabletten statt einer verpackt.

Bei einer von der Fa. ERWEXA Apparatebau GmbH offenkundig vorbenutzten Transporteinrichtung für Tabletten gemäß dem Oberbegriff des Schutzanspruchs 1 ist es nicht in jedem Falle gewährleistet, daß lediglich eine Tablette den Tabletten-Prüfstationen bzw. einer Verpackungseinrichtung zugeführt wird. Bei der bekannten Transporteinrichtung besteht die Zufuhreinrichtung für Tabletten aus einem Einfülltrichter, dessen Auslaß mit einer Rutsche in Verbindung steht, über die Tabletten zu der Transporteinrichtung gelangen können. Dabei verschließt eine Pendelklappe den Auslaß. Hierdurch soll erreicht werden, daß die Tabletten nacheinander den Auslaß passieren und über die Rutsche zu der Transporteinrichtung gelangen. Unter Umständen kann es jedoch vorkommen, daß zwei Tabletten auf einmal durch die Pendelklappe hindurchtreten, gemeinsam auf die Transporteinrichtung fallen und von einer V-förmigen Gabel der Transporteinrichtung nebeneinander liegend beispielsweise zu einer Waage transportiert werden.

Der Erfindung liegt die Aufgabe zugrunde, eine Transporteinrichtung für Tabletten gemäß dem Oberbegriff des Schutzanspruchs 1 derart weiterzubilden, daß unter allen Umständen sichergestellt ist, daß in einem Arbeitsgang lediglich eine Tablette von der Transporteinrichtung transportiert wird.

Diese Aufgabe wird erfindungsgemäß durch die im kennzeichnenden Teil des Schutzanspruchs 1 angegebenen Merkmale gelöst. Dabei liegt der Erfindung die Erkenntnis zugrunde, daß eine Tablette immer im Bereich der Spitze des "V" des Rechens anliegt, während zwei gleichzeitig auf die Führungsbahn gelangte Tabletten nebeneinander liegend transportiert werden, so daß die Tabletten an der V-förmigen Gabel nur im Bereich dessen Seitenwände anliegen. Anders ausgedrückt, werden immer dann, wenn mehr als eine Tablette gleichzeitig transportiert wird, die Tabletten mit Ausnahme höchstens einer Tablette außerhalb des Bereichs transportiert, in die der V-förmige Gabel eine einzelne Tablette transportiert.

Durch die erfindungsgemäßaußerhalb der Führungsbahn, und zwar beidseitig der Führungsbahn vorgesehenen Öffnungen fallen die überzähligen Tabletten von der Führungsbahn. Die Öffnungen sind dabei so dimensioniert, daß eine Tablette, die an der Spitze des "V" des Rechens anliegt, sicher zwischen den Öffnungen hindurch transportiert wird.

Weiterbildungen der Erfindung sind in den Unteransprüchen angegeben.

Gemäß Anspruch 3 sind die Öffnungen in der Führungsbahn über eine Rutsche mit einem Sammelbehälter verbunden. Damit können die nicht von der Transporteinrichtung weiter transportierten Tabletten beispielsweise erneut über die Zufuhreinrichtung der Transporteinrichtung zugeführt werden.

Die unsymmetrische Anordnung der Öffnungen in Verbindung mit der Verengung des Durchlasses zwischen den Öffnungen in Transportrichtung verhindert, daß dann, wenn zwei Tabletten gleichzeitig transportiert werden, beide Tabletten durch die Öffnungen von der Führungsbahn fallen, und so keine Tablette beispielsweise einer Verpackungseinrichtung zugeführt wird (Anspruch 4).

Die Erfindung wird nachstehend anhand eines Ausführungsbeispiels unter Bezugnahme auf die Zeichnung näher beschrieben, in der zeigen:

Fig. 1 eine zum Teil aufgebrochene Ansicht eines Tabletten-Prüfgeräts mit einer erfindungsgemäß Transporteinrichtung,

Fig. 2 einen Querschnitt durch das in Fig. 1 gezeigte Gerät (bei II-II in Fig.3), und

Fig. 3 eine Aufsicht auf das in Fig. 1 gezeigte Gerät.

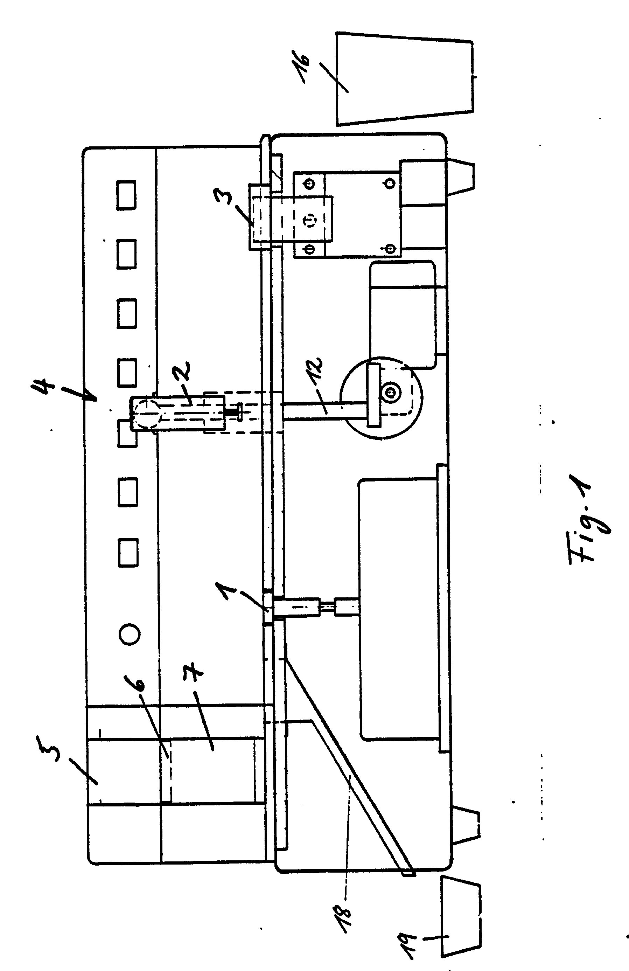

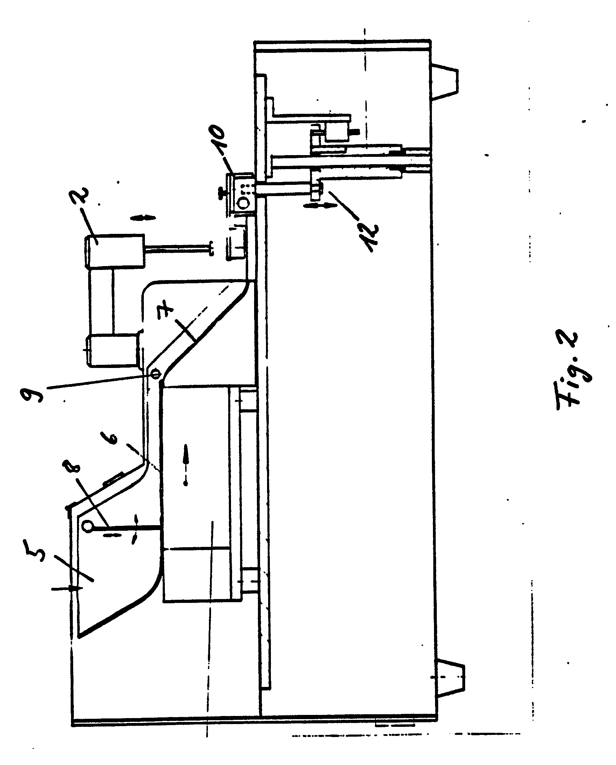

Das in den Fig. 1, 2 und 3 dargestellte Tabletten-Prüfgerät weist in an sich bekannter Weise eine Waage 1, eine Dickenmeßeinrichtung 2 sowie ein Bruchmesser 3 zum Zertrümmern der Tabletten auf. Die verschiedenen Prüfstationen sowie der Transport der Tabletten zwischen den Prüfstationen werden von einer nicht dargestellten elektronischen Steuerung, beispielsweise einer Mikroprozessor-Steuerung gesteuert, die über Tasten 4 bedient wird.

Die Waage 1, die Dickenmeßeinrichtung 2 sowie das Bruchmesser 3 sind bekannt, so daß auf ihre Funktion im Folgenden genausowenig eingegangen werden muß, wie auf die Funktion der elektronischen Steuerung, die jederzeit realisierbar ist.

Das erfindungsgemäß ausgebildete Tabletten-Prüfgerät weist ferner eine Zufuhreinrichtung für die Tabletten und eine Transporteinrichtung auf, die die Tabletten von einer Prüfstation zur nächsten transportiert.

Die Zufuhreinrichtung besteht aus einem Einfülltrichter 5, dessen Boden ein Linearförderer 6, beispielsweise ein kleines Fließband bildet. An den Linearförderer 6 schließt sich eine Rutsche 7 an. Ferner weist die Zufuhreinrichtung eine Pendelklappe 8 auf, die im Bereich der Austrittsöffnung des Einfülltrichters 5 über dem Linearförderer 6 angebracht ist. Im Bereich der Rutsche ist ferner eine Lichtschranke 9 angeordnet, mit der die Förderung einer Tablette erkannt und die elektronische Steuerung entsprechend gesteuert wird.

Die Transporteinrichtung weist in an sich bekannter Weise einen Rechen 10 mit mehreren V-förmigen Cabeln $11_1$, $11_2$, ... $11_6$ auf. Eine Bewegungsvorrichtung für den Rechen transportiert den Rechen 10 mit einem Exzenter 12 um eine Strecke d in Richtung eines Pfeils 13, hebt dann den Rechen I4 an und führt ihn im angehobenen Zustand entgegen der Richtung des Pfeils 13 um die Strecke d zurück. Anschließend wird der Rechen abgesenkt und erneut um die Strecke d in Richtung des Pfeils 13 verschoben.

Hierdurch werden Tabletten 14, die von der Zufuhreinrichtung in die Führungsbahn 15 des Rechens 10 befördert werden, intermittierend zu den einzelnen Prüfstationen, d.h. zu der Waage 1, der Dickenmeßeinrichtung 2 bzw. dem Bruchmesser 3 und anschließend zu einem Abfallbehälter 16 transportiert.

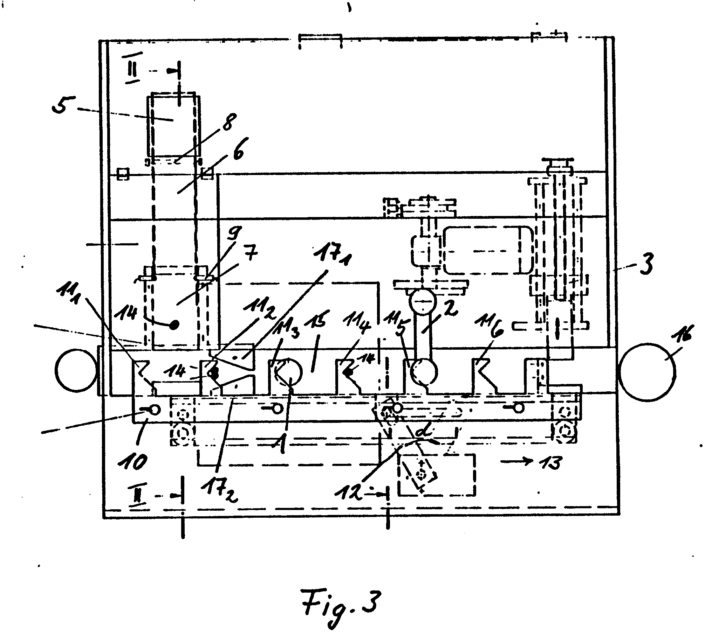

Vor der ersten Prüfstation, d.h. der Waage 1, sind beidseitig der Führungsbahn 15 Öffnungen $17_1$ bzw. $17_2$ angebracht. Die Öffnungen vergrößern sich in Transportrichtung der Tabletten hin zu der Führungsbahn. Der minimale Abstand zwischen den Öffnungen ist dabei so bemessen, daß Tabletten mit dem maximalen Durchmesser, der geprüft werden soll, sicher zwischen den Öffnungen hindurchtransportiert werden, wenn sie in der Spitze der V-förmigen Gabel 11 des Rechens anliegen. Tabletten, die nich an der Spitze anliegen, fallen dagegen in die Öffnungen $17_1$ bzw. $17_2$ und werden von dort über eine Überlaufrinne 18 zu einem Überlaufbehälter 19 befordert. Dies ist immer dann der Fall, wenn mehr als eine Tablette auf einmal von einer Gabel 11 befördert wird. Die geometrische Konfiguration, in der zwei Tabletten von einer Gabel befördert werden, ist beispielsweise an der Gabel $11_2$ dargestellt. Wie man aus Fig. 3 deutlich erkennt, werden beim Passieren der Öffnungen $17_1$ und $17_2$ die beiden Tabletten von der Führungsbahn abgestreift und fallen in die Öffnungen, so daß keine Tablette zu den Prüfstationen gelangt. Sofern dies unerwünscht sein sollte, können die öffnungen $17_1$ und $17_2$ auch unsymmetrisch ausgebildet werden. Hierdurch wird zunachst eine Tablette von der Führungsbahn abgestreift, wodurch die verbliebene Tablette zur Spitze der V-förmigen Gabel gelangt und so auf der Führungsbahn zwischen den Öffnungen die Öffnungen sicher passiert.

## Patentansprüche

1. Transporteinrichtung für Tabletten, die von einer Zufuhreinrichtung zugeführte Tabletten (14) mittels eines Rechens (10) mit mindestens einer V-förmigen Gabel (11) auf einer horizontalen Führungsbahn (15) zu Tabletten-Prüfstationen (1, 2, 3), Verpackungseinrichtung etc. transportieren, dadurch gekennzeichnet, daß die Führungsbahn (15) beidseitig des von der Spitze der V-förmigen Gabel (11) überstrichenen Bereichs Öffnungen ($17_1$, $17_2$) aufweist, deren minimaler Abstand so bemessen ist, daß Tabletten (14) mit dem maximalen Durchmesser, der geprüft werden soll, sicher zwischen den Öffnungen ($17_1$, $17_2$) hindurchtransportiert werden, wenn sie an der Spitze der V-förmigen Gabel (11) des Rechens (10) anliegen, wobei die Tabletten (14), die beim Transport nicht an der Spitze der V-förmigen Gabel (11) anliegen, in die Öffnungen ($17_1$, $17_2$) fallen.

2. Transporteinrichtung nach Anspruch 1, dadurch gekennzeichnet, daß die Öffnungen ($17_1$, $17_2$) sich in Transportrichtung der Tabletten (14) zu der Führungsbahn hin vergrößern.

3. Transporteinrichtung nach Anspruch 1 oder 2, dadurch gekennzeichnet, daß die Öffnungen ($17_1$, $17_2$) in der Führungsbahn über eine Rutsche (18) mit einem Sammelbehälter (19) verbunden sind.

4. Transporteinrichtung nach Anspruch 1, 2 oder 3, dadurch gekennzeichnet, daß die Öffnungen ($17_1$, $17_2$) unsymmetrisch ausgebildet sind und sich in Transportrichtung (13) auf die Führungsbahn (15) hin verbreitern.

## Claims

1. Transport unit for transporting tablets (14) fed by a filling unit by means of a rake (10) having at least one V-shaped fork (11) on a horizontal guiding track (15) to tablet testing stations (1, 2, 3), packing units etc., characterised in that the guiding track (15) comprises openings ($17_1$, $17_2$) on either side of the area swept by the point of the V-shaped fork (11), the minimum distance between said openings being dimensioned in a way that tablets (14) with the maximum diameter to be tested are safely transported between the openings ($17_1$, $17_2$) when they are disposed at the point of the V-shaped fork (11) of the rake (10) whereas tablets not disposed at said point of the V-shaped fork (11) during transport fall through the openings ($17_1$, $17_2$).

2. Transport unit according to claim 1, characterised in that the openings ($17_1$, $17_2$) increase in the transport direction of the tablets (14) towards the guiding track.

3. Transport unit according to claim 1 or 2, characterised in that the openings ($17_1$, $17_2$) in the guiding track are connected with a collector receptacle (19) via an inclined plane (18).

4. Transport unit according to claim 1, 2 or 3, characterised in that the openings ($17_1$, $17_2$) are made unsymmetrical and increase in the transport direction (13) towards the guiding track

(15).

## Revendications

1. Installation de transport pour des comprimés qui, à l'aide d'un râteau (10) ayant au moins une fourche en V (11), transporte des comprimés (14) amenés par un dispositif d'amenage sur une glissière horizontale de guidage (15) à des stations d'essai (1, 2, 3) pour comprimés, à des dispositifs d'emballage etc.,

caractérisée en ce que la glissière de guidage (15) possède, de tous les deux côtés du domaine balayé par la pointe de la fourche en V (11), des ouvertures ($17_1$, $17_2$) dont la distance minimum est déterminée de manière que les comprimés (14) ayant le diamètre maximum à essayer soient transportes infailliblement entre les ouvertures ($17_1$, $17_2$) quand ils adhèrent à la pointe de la fourche en V (11) du rateau (10), tandis que les comprimes (14) qui durant le transport n'adhèrent pas a la pointe de la fourche en V (11) tombent à travers les ouvertures ($17_1$, $17_2$).

2. Installation de transport selon la revendication 1, caractérisée en ce que les ouvertures ($17_1$, $17_2$) s'élargissent dans le sens de transport des comprimés (14) vers la glissière de guidage.

3. Installation de transport selon la revendication 1 ou 2, caractérisée en ce que les ouvertures ($17_1$, $17_2$) dans la glissière de guidage sont en communication avec un récipient collecteur (19) par l'intermédiaire d'un glissoir (18).

4. Installation de transport selon la revendication 1, 2 ou 3, caractérisée en ce que les ouvertures ($17_1$, $17_2$) sont dissymétriques et s'élargissent dans le sens de transport (13) vers la glissière de guidage (15).

Fig. 1

0170670

Fig. 2

Fig. 3